# FASCICULE DE BREVET EUROPEEN

(11) **EP 0 617 932 B1**
(45) Date de publication et mention de la délivrance du brevet: **27.05.1998**
(21) Numéro de dépôt: 94420107.8
(22) Date de dépôt: 29.03.1994
(51) Int. Cl.: A61F 2/34

(54) **Prothèse cotyloidienne élastiquement déformable**
Elastisch verformbare Hüftgelenkspfanneprothese
Elastically deformable acetabular cup prosthesis

(30) Priorité: 30.03.1993 FR 9303949
(43) Date de publication de la demande: 05.10.1994
(73) Titulaire: TORNIER SA, F-38330 Saint Ismier (FR)
(72) Inventeur: Carret, Jean-Paul, F-69190 Saint-Fons (FR); Fischer, Jean-Louis, F-69160 Tassin (FR); Chatelet, Jean-Christophe, F-69002 Lyon (FR); Fessy, Michel-Henri, F-69006 Lyon (FR); Bonnin, Michel, F-69110 Sainte-Foy-les-Lyon (FR); Nove-Josserand, Laurent, F-69006 Lyon (FR); Bejui, Jacques, F-69006 Lyon (FR); Galland, Olivier, F-38240 Meylan (FR)
(74) Mandataire: Karmin, Roger

(56) Documents cités:
- EP-A- 0 331 622
- EP-A- 0 353 171
- EP-A- 0 388 745
- EP-A- 0 453 694
- EP-A- 0 483 023
- EP-A- 0 498 685
- EP-A- 0 500 477
- WO-A-92/22265
- FR-A- 2 620 022
- US-A- 4 828 565

## Description

La présente invention est relative à une prothèse cotyloïdienne élastiquement déformable comportant un cotyle ou une calotte hémisphérique creuse en métal, en composite ou analogue qui est stabilisé dans l'os par des vis et un insert en plastique qui vient s'encliqueter à l'intérieur de ladite calotte.

On connaît des prothèses cotyloïdiennes de ce genre dans lesquelles la paroi externe de la calotte hémisphérique creuse est découpée d'une série de fentes d'expansion. Ces fentes sont réalisées en direction du centre de la calotte et l'une d'elles au moins débouche dans un trou, excentré ou non, délimitant ainsi une partie de calotte en plusieurs segments. Cette segmentation donne une certaine souplesse à la calotte hémisphérique creuse et facilite sa mise en place dans le cotyle humain.

De telles prothèses cotyloïdiennes comportent certains inconvénients en ce qui concerne la diminution du diamètre interne de la calotte hémisphérique creuse lors de la mise en place à l'intérieur de la cavité du cotyle humain. En effet, les fentes d'expansion donnent une certaine souplesse à la calotte, mais réduisent le diamètre interne de celle-ci, ce qui rend difficile, voire même impossible dans certain cas, la mise en place de l'insert plastique.

Les caractéristiques du préambule de la revendication 1 sont connues des documents FR-A-2620022 et WO-A-92/22265. WO-A-92/22265 divulgue une prothèse cotyloïdienne dans laquelle un cotyle est constitué d'une coque extérieure et d'une coque intérieure incompressible. Deux pièces sont nécessaires pour réaliser le cotyle et un système de fixation spécifique doit être prévu.

C'est à ces inconvénients qu'entend plus spécialement remédier la présente invention.

La prothèse cotyloïdienne suivant l'invention comporte un cotyle ou une calotte hémisphérique creuse en métal, en composite ou analogue, un insert en matière plastique telle que du polyéthylène, la calotte hémisphérique creuse comportant des moyens de déformation pour que la face externe de la calotte hémisphérique creuse se déforme élastiquement, tandis que sa partie interne reste rigide et indéformable. Elle est caractérisée en ce que les moyens de déformation consistent en des premières fentes et des deuxièmes fentes qui sont pratiquées dans l'épaisseur de la calotte de manière à ce que chacune desdites premières et deuxièmes fentes vien déboucher sur la face externe et sur le bord de celle-ci, tandis que deux desdites deuxièmes fentes se prolongent perpendiculairement à l'une de leurs extrémités libres sous la forme d'une rainure s'étendant vers l'extérieur de la calotte afin de définir deux ailes souples.

En outre, la prothèse cotyloïdienne comporte un insert en matière plastique qui est pourvu d'une cavité centrée sur l'axe de symétrie de ladite prothèse, et un chanfrein interne incliné d'un angle α par rapport à l'axe de symétrie afin de définir une portion de cylindre interne.

Le dessin annexé, donné à titre d'exemple, permettra de mieux comprendre l'invention, les caractéristiques qu'elle présente et les avantages qu'elle est susceptible de procurer :
Fig. 1 est une vue en perspective illustrant la prothèse cotyloïdienne suivant l'invention avant montage et plus particulièrement la calotte hémisphérique creuse et l'insert plastique.
Fig. 2 est une coupe suivant II-II (fig. 1) représentant la prothèse cotyloïdienne après montage de l'insert plastique dans la calotte hémisphérique creuse.

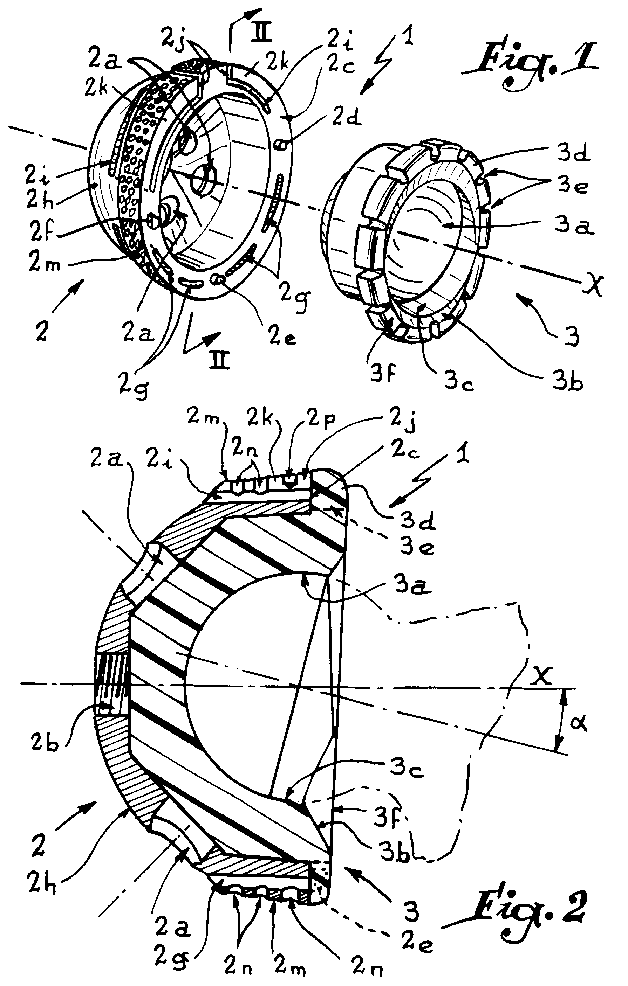

On a représenté en fig. 1 et 2 une prothèse cotyloïdienne 1 comportant un cotyle ou calotte 2 réalisé en titane ou analogue, et un insert 3 prévu en matière plastique telle que du polyéthylène.

La calotte hémisphérique 2 comprend, sur une partie de sa face externe 2h, un ensemble de trous débouchants 2a disposés suivant des rayons différents et permettant le passage de vis de fixation. Le fond de la calotte 2, et plus particulièrement son centre, est percé d'un trou débouchant et taraudé 2b permettant la fixation d'un ancillaire pour l'introduction de ladite calotte dans le cotyle humain.

Le bord circulaire 2c de la calotte 2 est pourvu de trois tétons 2d, 2e et 2f qui sont également répartis sur la moitié du pourtour dudit bord 2c. En fait, les tétons 2d et 2f sont alignés suivant l'un des axes de symétrie de la calotte 2, tandis que le téton 2e est orienté suivant l'autre axe de symétrie, c'est-à-dire à 90° des deux autres tétons.

Sur le bord 2c et entre les tétons 2d et 2e et les tétons 2e et 2f sont pratiquées des fentes 2g qui débouchent sur la face externe 2h de la calotte 2. De même, à l'opposé des fentes 2g et entre les tétons 2d et 2f sont percées deux fentes 2i qui débouchent sur la face externe 2h de la calotte. Les extrémités libres des fentes 2i qui se trouvent en vis-à-vis se prolongent perpendiculairement par une rainure 2j qui s'étend vers l'extérieur de la calotte 2 de manière à constituer deux ailes souples et élastiques 2k. On remarque que sur la face externe 2h de la calotte 2, les fentes 2g et 2i viennent déboucher au-dessous de la première rangée de trous 2a.

De même, entre le bord 2c et la première rangée de trous 2a est prévue une zone 2m qui permet au cal osseux de s'accrocher sur la face externe 2h de la calotte. Cette zone 2m est constituée d'une série de trous 2n qui débouchent dans les fentes 2g et 2i et une série de trous non débouchants 2p qui donnent un aspect particulier à la surface. Les trous 2n qui débouchent dans les fentes 2g et 2i permettent de créer un système complexe de chambres et de canaux sur la périphérie de la face externe 2h de la calotte 2 pour assurer une meilleure reprise et vascularisation du cal osseux. Cette reprise particulière du cal osseux permet une stabilité secondaire de la prothèse cotyloïdienne 1 de type biologique et non plus de type mécanique.

Dans l'insert plastique 3 est ménagée une cavité 3a qui est portée par le même axe de symétrie X de la prothèse cotyloïdienne 1 (fig. 2). La cavité 3a comporte un chanfrein interne 3b qui est incliné suivant un angle α d'environ 15° par rapport à l'axe X de la prothèse 1. L'inclinaison du chanfrein 3b à l'intérieur de la cavité 3a définit une portion de cylindre 3c qui permet d'éviter certains types de luxation. En effet, lorsque la boule céphalique humaine ou artificielle est introduite dans la cavité 3a de l'insert 3, il arrive parfois que sous un effort trop important, ladite boule céphalique s'échappe de la prothèse cotyloïdienne. Ainsi, la portion de cylindre 3c de l'insert 3 est prévue pour éviter ce genre de contrainte et de désagrément au patient.

L'insert 3 est pourvu d'une collerette 3d qui est percée sur son pourtour d'une série d'encoches 3e destinées à coopérer avec les tétons 2d, 2e et 2f de la calotte 2 lorsque l'insert 3 est introduit à l'intérieur de cette dernière.

La collerette extérieure 3d est prévue d'une épaisseur variable formant ainsi une face extérieure 3f inclinée afin que, par simple rotation de l'insert 3 dans la calotte 2, l'on puisse faire changer de plan ladite face suivant chaque cas pathologique.

On constate que le diamètre extérieur de la calotte hémisphérique 2 peut varier lors de son introduction dans la cavité humaine afin d'éviter certaines contraintes gênantes pour le patient, sans pour cela déformer son diamètre interne. La non-déformation du diamètre interne de la calotte hémisphérique creuse 2 permet une mise en place parfaite et facile de l'insert 3.

## Revendications

1. Prothèse cotyloïdienne, comprenant un cotyle ou calotte hémisphérique creuse en métal, en composite ou analogue, un insert en matière plastique telle que du polyéthylène, la calotte hémisphérique creuse comportant des moyens de déformation pour que la face externe de la calotte hémisphérique creuse se déforme élastiquement tandis que sa partie interne reste rigide et indéformable, caractérisée en ce que les moyens de déformation consistent en des premières fentes (2g) et des deuxièmes fentes (2i) qui sont pratiquées dans l'épaisseur de la calotte (2) de manière à ce que chacune desdites premières et deuxièmes fentes vien déboucher sur la face externe (2h) et sur le bord (2c) de celle-ci, tandis que deux desdites deuxièmes fentes (2i) se prolongent perpendiculairement à l'une de leurs extrémités libres sous la forme d'une rainure (2j) s'étendant vers l'extérieur de la calotte afin de définir deux ailes souples (2k).

2. Prothèse suivant la revendication 1, caractérisée en ce que les fentes (2g, 2i) débouchent sur la face externe (2h) au-dessous d'une première rangée de trous débouchants (2a) permettant le passage de vis pour la fixation de la calotte (2) dans la cavité humaine.

3. Prothèse suivant la revendication 1, caractérisée en ce que le bord (2c) de la calotte (2) comporte trois tétons (2d, 2e et 2f) qui sont également répartis sur la moitié du pourtour de ladite calotte (2) de manière à ce que le premier téton (2d) et le deuxième téton (2f) soient alignés suivant l'un des axes de symétrie de ladite calotte, tandis que le troisième téton (2e) est porté par l'autre axe de symétrie.

4. Prothèse suivant la revendication 3, caractérisée en ce que les premières fentes (2g) sont comprises d'une part entre le premier téton (2d) et le troisième téton (2e) et d'autre part entre le troisième téton (2e) et le deuxième téton (2f) tandis que les deuxièmes fentes (2i) sont prévues entre le deuxième téton (2f) et le premier téton (2d).

5. Prothèse suivant la revendication 1, caractérisée en ce que la face externe (2h) comporte une zone (2m) pour la construction du cal osseux, laquelle zone est comprise entre le bord (2c) et la première rangée de trous débouchants (2a).

6. Prothèse suivant la revendication 5, caractérisée en ce que la zone (2m) comporte un certain nombre de trous (2n) qui débouchent dans les fentes (2g et 2i), et un certain nombre de trous (2p) non débouchants.

7. Prothèse suivant la revendication 1, caractérisée en ce que l'insert plastique (3) comporte une cavité (3a) centrée sur l'axe de symétrie (X) de la prothèse (1), et un chanfrein interne (3b) qui est incliné d'un angle α par rapport à l'axe (X) afin de constituer une portion de cylindre (3c) qui empêche la luxation de la boule céphalique humaine ou artificielle.

8. Prothèse suivant la revendication 7, caractérisée en ce que l'insert (3) comporte une collerette (3d) qui est découpée d'encoches (3e) et dont l'épaisseur varie pour faire changer de plan sa face extérieure inclinée (3f) par rotation dudit insert à l'intérieur de la calotte (2).

9. Prothèse suivant la revendication 8, caractérisée en ce que les encoches (3e) de la collerette (3d) coopèrent avec les tétons (2d, 2e et 2f) de la calotte (2) en vue de permettre l'encliquetage de l'insert (3) à l'intérieur de celle-ci.

10. Prothèse suivant la revendication 8, caractérisée en ce que l'angle α d'inclinaison du chanfrein interne (3b) est égal à 15° par rapport à l'axe (X) de la prothèse (1).

## Claims

1. Acetabular cup prosthesis, comprising a cup or hollow hemispherical cap made of metal, composite or similar, an insert made of plastic material such as polyethylene, the hollow hemispherical cap including means of deformation so that the outer face of the hollow hemispherical cap deforms elastically while its inner part remains rigid and non-deformable, characterized in that the means of deformation consist of first slots (2g) and second slots (2i) which are made in the thickness of the cap (2) in such a way that each of the said first and second slots opens out on the outer face (2h) and on the edge (2c) thereof, while two of the said second slots (2i) continue perpendicularly at one of their free ends in the form of a groove (2j) extending towards the outside of the cap in order to define two flexible wings (2k).

2. Prosthesis according to Claim 1, characterized in that the slots (2g, 2i) open out on the outer face (2h) below a first row of open holes (2a) permitting the passage of screws for fixing the cap (2) in the human cavity.

3. Prosthesis according to Claim 1, characterized in that the edge (2c) of the cap (2) includes three studs (2d, 2e and 2f) which are uniformly distributed around half the circumference of the said cap (2) in such a way that the first stud (2d) and the second stud (2f) are aligned along one of the axes of symmetry of the said cap, while the third stud (2e) is held by the other axis of symmetry.

4. Prosthesis according to Claim 3, characterized in that the first slots (2g) are located, on the one hand, between the first stud (2d) and the third stud (2e) and, on the other hand, between the third stud (2e) and the second stud (2f), while the second slots (2i) are provided between the second stud (2f) and the first stud (2d).

5. Prosthesis according to Claim 1, characterized in that the outer face (2h) includes a zone (2m) for the build-up of bone callus, which zone is located between the edge (2c) and the first row of open holes (2a).

6. Prosthesis according to Claim 5, characterized in that the zone (2m) includes a number of holes (2n) which open out in the slots (2g and 2i), and a number of blind holes (2p).

7. Prosthesis according to Claim 1, characterized in that the plastic insert (3) includes a cavity (3a) centred on the axis of symmetry (X) of the prosthesis (1), and an inner bevel (3b) which is inclined by an angle α in relation to the axis (X) in order to constitute a cylinder portion (3c) which prevents luxation of the human or artificial hip ball.

8. Prosthesis according to Claim 7, characterized in that the insert (3) includes a flange (3d) which is cut with notches (3e) and whose thickness varies so as to change the plane of its inclined outer face (3f) by rotating the said insert inside the cap (2).

9. Prosthesis according to Claim 8, characterized in that the notches (3e) of the flange (3d) cooperate with the studs (2d, 2e and 2f) of the cap (2) with a view to making it possible to snap the insert (3) inside the cap.

10. Prosthesis according to Claim 8, characterized in that the angle α of inclination of the inner bevel (3b) is equal to 15° in relation to the axis (X) of the prosthesis (1).

## Patentansprüche

1. Hüftgelenkspfannenprothese aus einer Gelenkpfanne oder einer halbkugelförmigen Hohlkalotte aus Metall, Verbundwerkstoff oder dergleichen, und aus einem Einsatzteil aus Kunststoff wie Polyethylen, wobei die halbkugelförmige Hohlkalotte solche Mittel zur Verformung aufweist, daß sich die Mantelfläche der halbkugelförmige Hohlkalotte elastisch verformen kann, während deren Innenbereich steif und unverformbar bleibt,
**gekennzeichnet** durch
Mittel Zur Verformung in Form von ersten Schlitzen (2g) und zweiten Schlitzen (2i), die so über die Wanddicke der Hohlkalotte (2) verlaufen, daß der dieser ersten und zweiten Schlitze sowohl auf die Mantelfläche (2h), als auch auf die Umrandung (2c) dieser Hohlkalotte münden, während sich zwei dieser zweiten Schlitze (2i) an einem ihrer freien Enden senkrecht zum Außenumfang der Hohlkalotte hin in der Form einer Einkerbung (2j) erstrecken, um so zwei elastische Flügel (2k) auszubilden.

2. Prothese nach Anspruch 1,
dadurch **gekennzeichnet,**
daß die Schlitze (2g, 2i) unterhalb einer ersten Reihe an Durchgangslöchern (2a) auf die Mantelfläche (2h) münden, die der Aufnahme von Schrauben zur Befestigung der Hohlkalotte (2) in der Gelenkhöhle des Patienten dienen.

3. Prothese nach Anspruch 1,
dadurch **gekennzeichnet,**
daß die Umrandung (2c) der Hohlkalotte (2) drei Zapfen (2d, 2e, 2f) aufweist, die jeweils so auf dem halben Umfang dieser Hohlkalotte (2) verteilt sind, daß der erste Zapfen (2d) und der zweite Zapfen (2f) entlang einer der Symmetrieachsen dieser Hohlkalotte ausgerichtet sind während der dritte Zapfen (2e) in der anderen Symmetrieachse liegt.

4. Prothese nach Anspruch 3,
dadurch **gekennzeichnet,**
daß sich die ersten Schlitze (2g) zum einen zwischen dem ersten Zapfen (2d) und dem dritten Zapfen (2e) und zum anderen zwischen dem dritten Zapfen (2e) und dem zweiten Zapfen (2f) befinden, während die zweiten Schlitze (2i) zwischen dem zweiten Zapfen (2f) und dem ersten Zapfen (2d) vorgesehen sind.

5. Prothese nach Anspruch 1,
dadurch **gekennzeichnet,**
daß die Mantelfläche (2h) zwischen der Umrandung (2c) und der ersten Reihe an Durchgangslöchern (2a) einen Abschnitt (2m) zur Aufnahme des Knochenkeimgewebes aufweist.

6. Prothese nach Anspruch 5,
dadurch **gekennzeichnet,**
daß der Abschnitt (2m) eine bestimmte Anzahl an Löchern (2n), die in die Schlitze (2g, 2i) münden, und eine bestimmte Anzahl an Sacklöchern (2p) aufweist.

7. Prothese nach Anspruch 1,
dadurch **gekennzeichnet,**
daß das Einsatzteil (3) aus Kunststoff einen um die Mittelachse (X) der Prothese (1) ausgebildeten Hohlraum (3a) und eine Innenfase (3b) aufweist, die in einem solchen Winkel α zur Mittelachse (X) geneigt ist, daß sich ein zylindrischer Bereich (3c) ausbildet, der das Ausrenken des menschlichen oder prothetischen Gelenkkopfes verhindert.

8. Prothese nach Anspruch 7,
dadurch **gekennzeichnet,**
daß das Einsatzteil (3) einen Flansch (3d) aufweist, der von Einschnitten (3e) unterteilt ist, und dessen Dicke variiert, so daß durch Drehen dieses Einsatzteils im Inneren der Hohlkalotte (2) die Lage der geneigten Außenfläche (3f) des Flansches geändert werden kann.

9. Prothese nach Anspruch 8,
dadurch **gekennzeichnet,**
daß die Einschnitte (3e) im Flansch (3d) mit den Zapfen (2d 2e, 2f) der Hohlkalotte (2) so zusammenwirken, daß das Einsatzteil (3) im Inneren dieser Hohlkalotte gehemmt ist.

10. Prothese nach Anspruch 8,
dadurch **gekennzeichnet,**
daß die Innenfase (3b) in einem Winkel α von 15° zur Mittelachse (X) der Prothese (1) geneigt ist.
